Europäisches Patentamt

European Patent Office

Office européen des brevets

(11) Publication number: **0 303 467**
**A2**

(12) **EUROPEAN PATENT APPLICATION**

(21) Application number: **88307401.5**

(22) Date of filing: **10.08.88**

(51) Int. Cl.⁴: **A 61 F 2/38**

(30) Priority: **10.08.87 US 83133**

(43) Date of publication of application:
**15.02.89 Bulletin 89/07**

(84) Designated Contracting States: **DE FR GB**

(71) Applicant: **DOW CORNING WRIGHT CORPORATION**
**P.O. Box 100 5677 Airline Road**
**Arlington Tennessee (US)**

(72) Inventor: **Lacey, James Allen**
**3 Isle of Sicily**
**Winter Park Florida (US)**

(74) Representative: **Lewin, John Harvey et al**
**ELKINGTON AND FIFE Beacon House 113 Kingsway**
**London WC2B 6PP (GB)**

(54) **Prosthesis for femoral component of knee joint.**

㊇ A prosthetic device is provided for replacing the distal articulating surfaces of a femur, implantable without bone cement, which includes a base portion having downwardly facing condylar surfaces, and unitary therewith, upwardly extending flanges which engage and compress the anterior and posterior femoral resection surfaces. The bone engaging flange surfaces are provided with a plurality of toothlike projections of a downwardly angled sawtooth configuration adapted to compress the resected bone and anchor the prosthesis thereto.

EP 0 303 467 A2

Description

## PROSTHESIS FOR FEMORAL COMPONENT OF KNEE JOINT

The knee is generally regarded as being the most inherently unstable of the joints of the human body, due in part to the complex inter-related types of motions to which the several knee elements are subjected during ambulatory movements of the body. Various prostheses have hithertofore been proposed in attempts to approximate, through an artificial joint, the natural action of the human knee. Advanced conditions of disease or serious traumatic injury of the knee joint complicate surgical repair and efforts to simulate the natural knee motion through use of a prosthesis.

Several types of prostheses, each having advantages and disadvantages have been employed. One type currently in use is the hinged type such as described in my U.S. Patent No.4,262,368. Other prostheses are employed in which the femoral and tibial components are unconnected. Most of the latter systems rely on the use of bone cement to assist in anchoring the prosthesis to the resected femur. Some prostheses, for example, that are shown in U.S. Patent No. 4,550,448 utilize porous surfaces in an attempt to achieve bone ingrowth for anchoring.

U.S. Patent No. 4,355,429 discloses a knee joint prosthesis which is secured without cement. Anchoring is accomplished by utilizing pins inserted into slightly undersize holes drilled into the bone. There is no disclosure of the use of individual projections integral with upwardly extending flanges of the implant which serve to mechanically anchor the device.

One commercially available implant, referred to as the Kodama-Yamamoto Total Knee uses a waffled interior surface to obtain anchoring to the bone along with anchoring staples. See "The Spectrum of Total Knee Replacement" Replacement of the Total Knee, Laskin-Verlag-Apley, Sprig-Verlag, New York, 1984, pp. 29-30. This system does not employ projections on the inner surfaces of the flanges having a downwardly angled sawtooth configuration This design is believed, instead, to rely on a tight mechanical fit of the prosthesis over the bone along with the hope that bone ingrowth into the waffle-like surface will occur.

The general objects of the present invention are to provide an improved femoral prosthesis designed for insertion without cement. The implants of the present invention provide immediate mechanical fixation to the bone rather than relying on bone ingrowth into a porous or textured surface as in the case of many prostheses currently available.

The invention provides a femoral implant prosthesis designed to replace the articulating surfaces of the human distal femur which is implanted without the use of bone cement. The prosthesis of this invention is a unitary one-piece device adapted to be mechanically connected to the resected distal end of the femur by upward driving implantation. The prosthesis comprises a base portion having downwardly facing condylar surfaces and upwardly extending anterior and posterior flanges integral with the base. The flanges are designed to engage and compress the anterior and posterior femoral resection surfaces. The bone engaging surfaces of said flanges are provided with a plurality of toothlike projections of a downwardly angled sawtooth configuration such that the implant is removed with significantly greater difficulty than it is applied. Also integral with the base is at least one protrusion extending upwardly from the central portion of the base. The preferred configuration for the protrusion is that of a C-shaped configuration such as that shown by Waugh et al. in U.S. Patent No. 3,869,731. Other configurations used in conjunction with appropriately cut or drilled slots or holes in the bone may also be employed.

Preferred embodiments of the invention will be set forth by way of example in the following detailed description and appended drawings, wherein:

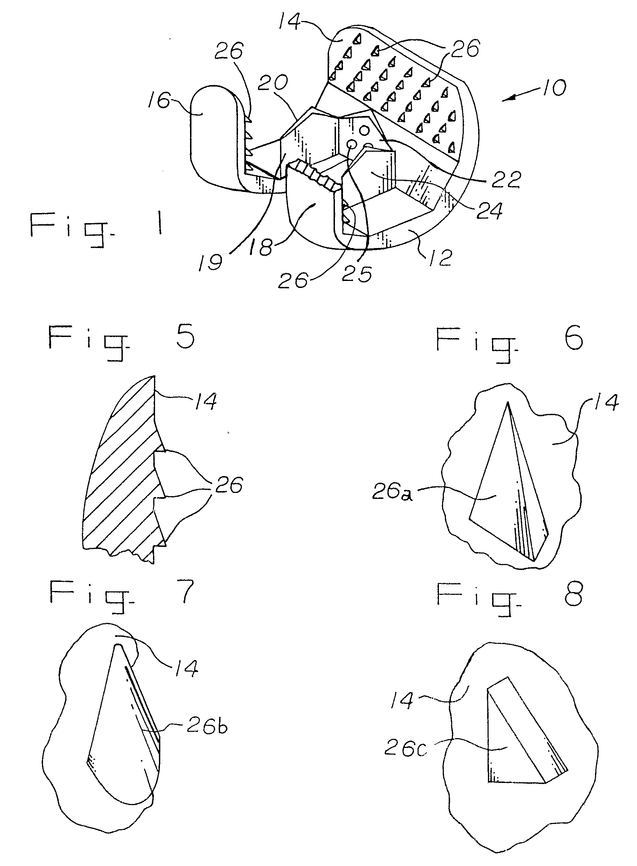

Fig. 1 is a lateral-posterior perspective view with parts broken away of a prosthesis according to the invention.

Fig. 2 is top view of said prosthesis.

Fig. 3 is a lateral view of the prosthesis.

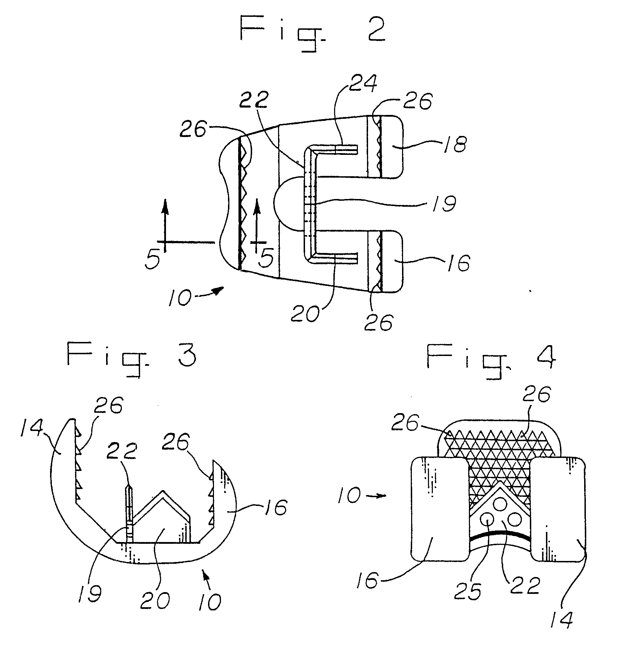

Fig. 4 is a posterior view of the prosthesis.

Fig. 5 is an enlarged broken away sectional view taken on line 5-5 of Fig. 2.

Fig. 6 is an enlarged, broken away perspective view showing one form of a tooth in accordance with the invention.

Fig. 7 is an enlarged, broken away perspective view showing another embodiment of a tooth which may be employed.

Fig. 8 is an enlarged, broken away perspective view showing yet another embodiment of the invention.

Referring specifically to the drawings, there is seen a unitary, one piece prosthesis identified generally by numeral 10, which is designed to replace the articulating surfaces of a human femur. The prosthesis or implant is preferably made of cobalt-chromium alloy or similar biocompatible and non-corroding material. The unitary construction includes a base portion 12 having downwardly facing condylar surfaces and also having integral thereto an upwardly extending anterior flange portion 14 and upwardly extending posterior flange portions 16 and 18. The outer surfaces of anterior flange portion 14 are curved to approximate the anterior femoral condylar surfaces of the Patient for articulation with the patella. Posterior flange portions 16 and 18 similarly have external surfaces curved to approximate the posterior femoral condylar surfaces of the patient for articulation with a natural or prosthetic tibia. Also, in the preferred embodiment, there is integral with the base portion 10 and extending upwardly from the central portion thereof an anchoring protrusion 19. As illustrated, the preferred configuration for protrusion 19 is that of a C-shape with individual sharpened fins 20, 22, and 24. The fins 20, 22, and 24 may each be provided with holes 25 which allow for bone ingrowth after implantation of the prosthesis.

Flanges 14, 16, and 18 are provided with approximately parallel inner surfaces adapted to engage the anterior and posterior surfaces of the resected femur. The inner surface of each of said flanges 14, 16, and 18 is Provided with a plurality of inwardly facing projections 26 of a downwardly angled configuration. Projections 26 are formed so that the upper surfaces thereof extend inwardly away from the surfaces of the flanges at a shallow angle with respect to the flange surface. i.e., approximately 10° to 35°. The lower surface of each Projection extends inwardly from the inner surface of the flange on which it is located at a steep angle with respect to the flange surface, i.e., approximately 75° to 105°. This configuration of the projections provides a construction which can be implanted over a slightly oversized resected distal end of a femur much more easily than it can be removed, in a fashion somewhat analogous to the action of an arrowhead. These protrusions, due to their shape, and the tight fit of the flanges over the bone provide immediate mechanical fixation of the prosthesis by compressing the bone. Any downward pulling force on the prosthesis will tend to anchor the projections more firmly into the bone. The configuration allows easy implantation of the Prosthesis and allows a natural distribution of stress distally, thereby promoting physiological loading, i.e., the load is transferred sufficiently to the resected distal surface to prevent atrophy of said surface over a period of time.

As shown in Figures 6 through 8, inclusive, the tooth-like projections 26 may be of a variety of configurations, indicated as 26a, 26b, and 26c. For example, the individual teeth may be of a triangular cross section, shown as 26a in Fig.6, elliptoid, shown as 26b in Fig.7, or rectangular or trapezoidal, shown as 26c in Fig.8.

In implantation, the bone is osteotomized to provide slightly undersized slots to receive anchoring protrusion 19 thus providing for further compression of the bone.

It is to be understood that the foregoing embodiments are to be considered illustrative of the invention. Various modifications, changes or alterations of the invention disclosed herein may be evident to those skilled in the art. Thus, the invention disclosed herein is not intended to be limited by the description hereinabove, but rather. is intended to be limited only by the appended claims.

## Claims

1. A prosthetic device for replacing at least a portion of the distal end of a femur comprising a unitary, one-piece device adapted to be connected to the distal end of a resected femur without cement and to replace the condylar surfaces thereof, said prosthesis including

(A) a base portion having downwardly facing condylar surfaces,

(B) upwardly extending anterior and posterior flanges integral with said base portion, said flanges being designed to engage and compress the anterior and posterior femoral resection surfaces, and

(C) the bone engaging surfaces of said flanges being provided with a plurality of toothlike projections of a downwardly angled sawtooth configuration, said projections being adapted to compress the resected bone and anchor said prosthesis thereto.

2. A femoral prosthesis according to claim 1 which is provided with an anchoring protrusion extending upwardly from the top of said base for connecting the prosthesis to the femur by upward driving implantation.

3. A prosthetic device for replacing at least a portion of the articulating surfaces of the distal end of a femur comprising a unitary, one-piece device adapted to be connected to the distal end of a resected femur without cement and to replace the condylar surfaces thereof, said prosthesis including

(A) a base portion having downwardly facing condylar surfaces,

(B) upwardly extending anterior and posterior flanges integral with said base portion, said flanges being designed to engage and compress the anterior and posterior femoral resection surfaces,

(C) the bone engaging surfaces of said flanges being provided with a plurality of projections of a downwardly angled configuration, said projections being adapted to compress the resected bone and anchor said prosthesis thereto, and having upper surfaces extending inwardly from said flange surfaces at a shallow angle relative thereto, and lower surfaces extending inwardly at a steep angle relative thereto, and

(D) an anchoring protrusion extending upwardly from the top of said base for connecting the prosthesis to the femur by upward driving implantation.

4. A femoral prosthesis according to claim 1 which is provided with an anchoring protrusion extending upwardly from the top thereof which has a C-shaped configuration.

Fig. 1

Fig. 5

Fig. 6

Fig. 7

Fig. 8

Fig. 2

Fig. 3

Fig. 4